# EUROPÄISCHE PATENTSCHRIFT

(11) **EP 1 294 731 B1**
(45) Veröffentlichungstag und Bekanntmachung des Hinweises auf die Patenterteilung: **18.02.2004**
(21) Anmeldenummer: 01956490.5
(22) Anmeldetag: 20.06.2001
(51) Int. Cl.: C07F 9/6574, C07F 9/144, C07F 9/143, C07F 9/142, C07F 9/145

(54) **BISPHOSPHITVERBINDUNGEN, DEREN METALLKOMPLEXE UND VERWENDUNG DER VERBINDUNGEN UND KOMPLEXE IN DER OLEFINHYDROFORMYLIERUNG**
BISPHOSPHITE COMPOUNDS, THE METAL COMPLEXES THEREOF AND THE USE OF SAID COMPOUNDS AND COMPLEXES IN OLEFIN HYDROFORMYLATION
COMPOSES BIPHOSPHITES, LEURS COMPLEXES METALLIQUES, ET UTILISATION DES COMPOSES ET DES COMPLEXES DANS L'HYDROFORMYLATION DES OLEFINES

(30) Priorität: 28.06.2000 DE 10031493
(43) Veröffentlichungstag der Anmeldung: 26.03.2003
(73) Patentinhaber: Oxeno Olefinchemie GmbH, 45772 Marl (DE)
(72) Erfinder: RÖTTGER, Dirk, 45657 Recklinghausen (DE); KADYROV, Renat, 65931 Frankfurt (DE); BÖRNER, Armin, 18059 Rostock (DE); SELENT, Detlef, 10318 Berlin (DE); HESS, Dieter, 45770 Marl (DE)
(86) Internationale Anmeldenummer: PCT/EP2001/006968
(87) Internationale Veröffentlichungsnummer: WO 2002/000670

(56) Entgegenhaltungen:
- EP-A- 0 577 042
- KADYROV R ET AL: "New carbohydrate bisphosphites as chiral ligands" TETRAHEDRON: ASYMMETRY, ELSEVIER SCIENCE PUBLISHERS, AMSTERDAM, NL, Bd. 9, Nr. 2, 30. Januar 1998 (1998-01-30), Seiten 329-340, XP004131180 ISSN: 0957-4166
- KONIG T ET AL: "SYNTHESEN UND NMR-SPEKTREN PHOSPHORORGANISCHER ANTIOXIDANTIEN UND VERWANDTER VERBINDUNGEN" JOURNAL FUER PRAKTISCHE CHEMIE, CHEMIKER ZEITUNG, WILEY VCH, WEINHEIM, DE, Bd. 334, Nr. 4, 1992, Seiten 333-349, XP000647405 ISSN: 1436-9966
- RUEGER C ET AL: "KINETIK UND MECHANISMUS DER CUMYLHYDROPEROXIDZERSETZUNG DURCH CYCLISCHE PHOSPHITE" JOURNAL FUER PRAKTISCHE CHEMIE, LEIPZIG, DE, Bd. 326, Nr. 4, 1984, Seiten 622-632, XP000646969 ISSN: 0021-8383
- SCHWETLICK, KLAUS ET AL: "Reaktionen trivaleneter Phosphorverbindungen mit tert-Butoxylradikalen" ZEITSCHRIFT FUER CHEMIE, Bd. 26, Nr. 10, 1986, Seiten 360-366, XP001018820 ISSN: 0044-2402
- RUZAEVA, M. I. ET AL: "Synthesis and chemical specifics of bicyclophosphites and bicyclophosphoramidites derived from anthracene-1,4,9,10-tetraol" RUSSIAN JOURNAL OF GENERAL CHEMISTRY, Bd. 70, Nr. 9, 2000, Seiten 1363-1367, XP001029356 ISSN: 1070-3632
- NIFANT'EV, E. E. ET AL: "Synthesis and investigation of 1,8- and 1,2-naphthylene phosphites" JOURNAL OF GENERAL CHEMISTRY OF THE USSR, Bd. 51, Nr. 7, 1981, Seiten 1295-1299, XP001029125 ISSN: 0022-1279
- NIFANT'EV, E. E. ET AL: "Synthesis of 1,8-naphythylene phosphites" JOURNAL OF GENERAL CHEMISTRY OF THE USSR, 49(10), 2390 , Bd. 49, Nr. 10, 1979, Seite 2109 XP001029122 ISSN: 0022-1279
- KOENIG, T. ET AL: "Phosphororganische Antioxidanten. X. Über die hydroperoxidzersetzende Wirkung von Phosphiten, Phosphoniten und Thiophosphiten" JOURNAL FUER PRAKTISCHE CHEMIE, Bd. 331, Nr. 6, 1989, Seiten 913-922, XP001029735 ISSN: 0021-8383
- VOROPAI, L. M. ET AL: "2,3-Naphthylene phosphites" JOURNAL OF GENERAL CHEMISTRY OF THE USSR, Bd. 55, Nr. 1, 1985, Seiten 55-62, XP001029750 ISSN: 0022-1279
- RUEGER, C. ET AL: "Phosphororganische Antioxidanten. VI. Enifluss cyclischer Phosphite auf die radikalisch initiierte Oxidation von Kohlenwasserstoffen und Polymeren" ACTA POLYMERICA, Bd. 37, Nr. 7, 1986, Seiten 435-438, XP001029738 ISSN: 0323-7648
- SCHWETLICK, K. ET AL: "Chain-breaking antioxidant activity of phosphite esters" POLYMER DEGRADATION AND STABILITY, Bd. 15, Nr. 2, 1986, Seiten 97-108, XP001029355 ISSN: 0141-3910

## Beschreibung

Die vorliegende Erfindung betrifft Bisphosphite und deren Metallkomplexe, die Herstellung, sowie die Verwendung der Bisphosphite als Ligand in katalytischen Reaktionen.

Die Reaktionen zwischen Olefinverbindungen, Kohlenmonoxid und Wasserstoff in Gegenwart eines Katalysators zu den um ein C-Atom reicheren Aldehyden ist als Hydroformylierung (Oxierung) bekannt. Als Katalysatoren in diesen Reaktionen werden häufig Verbindungen der Übergangsmetalle der VIII. Gruppe des Periodensystems der Elemente verwendet, insbesondere Rhodium und Kobaltverbindungen. Die Hydroformylierung mit Rhodiumverbindungen bietet im Vergleich zur Katalyse mit Kobalt in der Regel den Vorteil höherer Selektivität und ist damit meistens wirtschaftlicher. Bei der durch Rhodium katalysierten Hydroformylierung werden zumeist Komplexe eingesetzt, die aus Rhodium und bevorzugt aus trivalenten Phosphorverbindungen als Ligahden bestehen. Bekannte Liganden sind beispielsweise Verbindungen aus den Klassen der Phosphine, Phosphite und Phosphonite. Eine gute Übersicht über den Stand der Hydroformylierung von Olefinen findet sich in B. CORNILS, W. A. HERRMANN, *"Applied Homogeneous Catalysis with Organometallic Compounds",* Vol. 1&2, VCH, Weinheim, New York, 1996.

Jedes Katalysatorsystem (Kobalt oder Rhodium) hat seine spezifischen Vorzüge. Je nach Einsatzstoff und Zielprodukt kommen daher unterschiedliche Katalysatorsysteme zum Einsatz, wie folgende Beispiele zeigen. Arbeitet man mit Rhodium und Triphenylphosphin, lassen sich α-Olefine bei niedrigeren Drücken hydroformylieren. Als Phosphor-haltiger Ligand wird in der Regel Triphenylphosphin im Überschuss verwendet, wobei ein hohes Ligand/Rhodium-Verhältnis erforderlich ist, um die Selektivität der Reaktion zum kommerziell erwünschten n-Aldehydprodukt zu erhöhen.

Die Patente US 4 694 109 und US 4 879 416 beschreiben Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten erreicht. In WO 95/30680 werden zweizähnige Phosphinliganden und ihr Einsatz in der Katalyse, unter anderem auch in Hydroformylierungsreaktionen, offen gelegt. Ferrocenverbrückte Bisphosphine werden beispielsweise in den Patentschriften US 4 169 861, US 4 201 714 und US 4 193 943 als Liganden für Hydroformylierungen beschrieben.

Der Nachteil von zweizähnigen Phosphinliganden ist ein relativ hoher Aufwand, der zu ihrer Darstellung notwendig ist. Daher ist es oftmals nicht rentabel, solche Systeme in technischen Prozessen einzusetzen.

Rhodium-Monophosphit-Komplexe sind geeignete Katalysatoren für die Hydroformylierung von verzweigten Olefinen mit innenständigen Doppelbindungen, jedoch ist die Selektivität für endständig oxierte Verbindungen gering. Aus EP 0 155 508 ist die Verwendung von bisarylensubstituierten Monophosphiten bei der rhodiumkatalysierten Hydroformylierung von sterisch gehinderten Olefinen, z. B. Isobuten bekannt. Monophosphite, die eine 1,8-Naphtylphosphitgruppierung aufweisen, sowie deren Herstellung werden unter anderem von König, T. et al., Journal für praktische Chemie, Chemiker Zeitung, Bd. 334, Nr. 4, 1992, Seiten 333-349, Rüger, C. et al., Journal für praktische Chemie, Bd. 326, Nr. 4, 1984, Seiten 622-632, Schwetlick, Klaus et al., Zeitschrift für Chemie, Bd. 26, Nr. 10, 1986, Seiten 360-366, Ruzaeva, M. I. et al., Russian Journal of General Chemistry, Bd. 70, Nr. 9, 2000, Seiten 1363-1367, Nifant'ev, E. E. et al., Journal of General Chemistry of the USSR, Bd. 51, Nr. 7, 1981, Seiten 1295-1299, Nifant'ev, E. E. et al., Journal of General Chemistry of the USSR, 49(10), 2390, Bd. 49, Nr. 10, 1979, Seite 2109, König, T. et al., Journal für praktische Chemie, Bd. 331, Nr. 6, 1989, Seiten 913-922, Voropai, L. M. et al., Journal of General Chemistry of the USSR, Bd. 55, Nr. 1, 1985, Seiten 55-62, Rüger, C. et al., Acta Polymerica, Bd. 37, Nr. 7, 1986, Seiten 435-438 und Schwetlick, Klaus et al., Polymer Degradation and Stability, Bd. 15, Nr. 2, 1986, Seiten 97-108 beschrieben.

Rhodium-Bisphosphit-Komplexe katalysieren die Hydroformylierung von linearen Olefinen mit end- und innenständigen Doppelbindungen, wobei überwiegend endständig hydroformylierte Produkte entstehen, dagegen werden verzweigte Olefine mit innenständigen Doppelbindungen nur im geringen Maße umgesetzt. Diese Phosphite ergeben bei ihrer Koordination an ein Übergangsmetallzentrum Katalysatoren von gesteigerter Aktivität, doch ist das Standzeitverhalten dieser Katalysatorsysteme, unter anderem wegen der Hydrolyseempfindlichkeit der Phosphitliganden, unbefriedigend. Durch den Einsatz von substituierten Bisaryldiolen als Edukte für die Phosphitliganden, wie in EP 0 214 622 oder EP 0 472 071 beschrieben, konnten erhebliche Verbesserungen erreicht werden.

Der Literatur zufolge sind die Rhodiumkomplexe dieser Liganden äußerst aktive Hydroformylierungskatalysatoren für α-Olefine. In den Patenten US 4 668 651, US 4 748 261 und US 4 885 401 werden Polyphosphitliganden beschrieben, mit denen α-Olefine, aber auch 2-Buten mit hoher Selektivität zu den terminal oxierten Produkten umgesetzt werden können. Zweizähnige Liganden dieses Typs wurden auch zur Hydroformylierung von Butadien eingesetzt (US 5 312 996 bzw. EP 0 577 042). Die dort eingesetzten Liganden weisen zwei Biphenylphosphitgruppierungen auf.

Obgleich die genannten Bisphosphite sehr gute Komplexliganden für Rhodium-Hydroformylierungskatalysatoren sind, ist es wünschenswert, deren Wirksamkeit und Hydrolysebeständigkeit noch weiter zu verbessern.

Es wurde gefunden, dass Bisphosphite mit den allgemeinen Struktur I 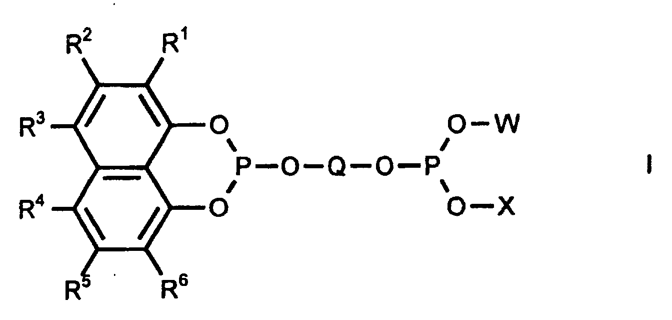 einfach hergestellt werden können und als Liganden bei Metall-katalysierten Reaktionen geeignet sind.

Gegenstand der vorliegenden Erfindung sind daher Bisphosphite der allgemeinen Formel I 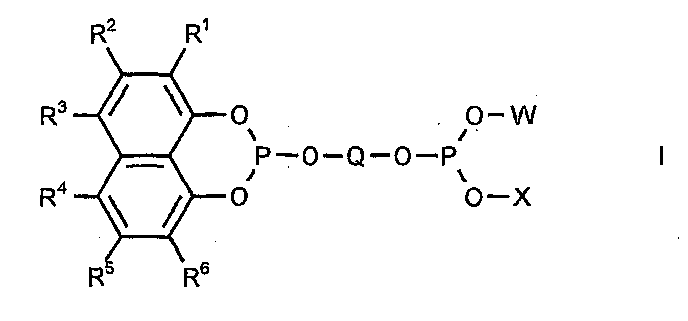 mit
R¹, R², R³, R⁴, R⁵, R⁶ = H, aliphatischer; alicyclischer, aliphatisch-alicyclischer, heterocyclischer, aliphatisch-heterocyclischer, aromatischer, aliphatisch-aromatischer Kohlenwasserstoffrest mit 1 bis 50 Kohlenstoffatomen, F, Cl, Br, I, -OR⁷, -COR⁷, -CO₂R⁷, -CO₂M, -SR⁷, -SO₂R⁷, -SOR⁷, -SO₃R⁷, -SO₃M, -SO₂NR⁷R⁸, NR⁷R⁸, N=CR⁷R⁸, NH₂, wobei R¹ bis R⁶ eine gleiche oder unterschiedliche Bedeutung besitzen und kovalent miteinander verknüpft sein können,
R⁷, R⁸ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion
Q = zweiwertiger aliphatischer, alicyclischer, aliphatisch-alicyclischer, heterocyclischer, aliphatisch-heterocyclischer, aromatischer, aliphatisch-aromatischer Kohlenwasserstoffrest mit 1 bis 50 Kohlenstoffatomen,
W, X = aliphatische, alicyclische, aliphatisch-alicyclische, heterocyclische, aliphatisch-heterocyclische, aromatische, aliphatisch-aromatische Kohlenwasserstoffreste mit 1 bis 50 Kohlenstoffatomen, die gleich oder unterschiedlich oder kovalent miteinander verknüpft sein können.

Spezielle Ausführungeformen der erfindungsgemäßen Bisphosphite betreffen Bisphosphite der Formeln II und III 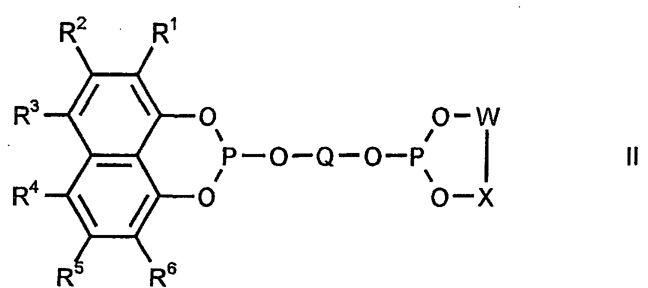 und 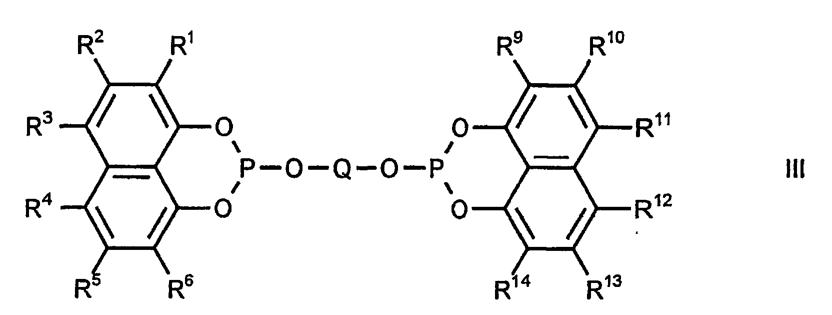 wobei W und X aliphatische, alicyclische, aliphatisch-alicyclische, heterocyclische, aliphatisch-heterocyclische, aromatische, aliphatisch-aromatische Kohlenwasserstoffreste mit 1 bis 50 Kohlenstoffatomen bedeuten, X und W gleich oder unterschiedlich oder kovalent mit einander verknüpft sein können
und R¹, R², R³, R⁴, R⁵, R⁶, R⁷, R⁸ und Q die bereits genannten Bedeutungen besitzen.

R⁹, R¹⁰, R¹¹, R¹², R¹³, R¹⁴ stehen für H, aliphatischer, alicyclischer, aliphatisch-alicyclischer, heterocyclischer, aliphatisch-heterocyclischer, aromatischer, aliphatisch-aromatischer Kohlenwasserstoffrest mit 1 bis 50 Kohlenstoffatomen, F, Cl, Br, l, -OR¹⁵, -COR¹⁵, -CO₂R¹⁵, -CO₂M, -SR¹⁵, -SO₂R¹⁵, -SOR¹⁵, -SO₃R¹⁵, -SO₃M, -SO₂NR¹⁵R¹⁶, NR¹⁵R¹⁶, N=CR¹⁵R¹⁶, NH₂, wobei R⁹ bis R¹⁴ eine gleiche oder unterschiedliche Bedeutung besitzen und kovalent miteinander verknüpft sein können.

M steht für ein Alkalimetall-, Erdalkalimetall-, Ammonium-, oder Phosphoniumion.

R¹⁵ und R¹⁶ können gleich oder unterschiedlich sein und jeweils für H, substituierte oder unsubstituierte, aliphatische oder aromatische Kohlenwasserstoffreste mit 1 bis 25 Kohlenstoffatomen, mit gleicher oder unterschiedlicher Bedeutung stehen.

Beispiele für Q sind bivalente Kohlenwasserstoffreste, die aliphatisch, alicyclisch, aliphatisch-alicyclisch, heterocyclisch, aliphatisch-heterocylisch, aromatisch oder aliphatisch-aromatisch sein können. Gegebenenfalls vorhandene Ringsysteme können ihrerseits mit den oben genannten Kohlenwasserstoffresten substituiert sein. In offenkettigen Strukturelementen können eine oder mehrere Methylengruppen durch Sauerstoff und/oder Schwefel und/oder NR⁷ und/oder NH und/oder eine oder mehrere CH-Gruppen durch Stickstoff ersetzt sein.

Bevorzugt steht Q für bivalente Reste, die aromatische Gruppen enthalten. Q kann beispielsweise ein Phenylenrest, Naphthylrest, ein zweiwertiger Bisarylenrest oder ein bivalenter Rest eines Diphenylethers sein. Weiterhin kann Q die allgemeine Struktur -Ar-Z-Ar- haben. Darin bedeutet Ar einen einringigen oder mehrringigen bivalenten aromatischen Rest. Z steht entweder für eine direkte Bindung oder für eine gegebenenfalls substituierte Methylengruppe -CR⁷R⁸-, wobei R⁷ und R⁸ für Wasserstoff und/oder aliphatische und/oder aromatische Reste mit 1 bis 25 Kohlenstoffatomen stehen und die darüber hinaus Heteroatome enthalten können. Weiterhin können die Reste R⁷ und R⁸ zu einem oder mehreren Ringen verknüpft sein, d. h. eine kovalente Bindung aufweisen.

Von den Bisphosphiten nach den allgemeinen Formeln I, II und III sind diejenigen besonders bevorzugt, bei denen der Rest Q für einen Kohlenwasserstoffrest (Bisarylenrest) nach der allgemeinen Formel IV steht 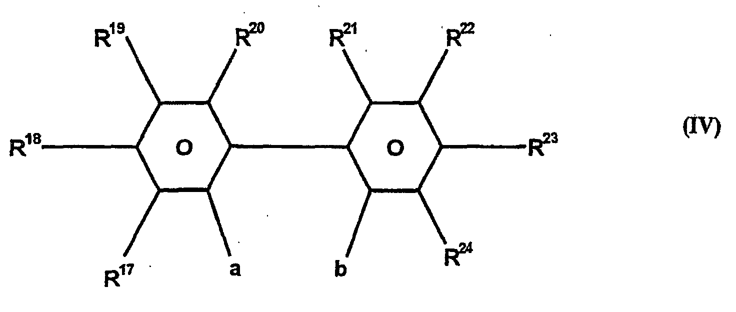 mit
R¹⁷, R¹⁸, R¹⁹, R²⁰, R²¹, R²², R²³, R²⁴ = H, aliphatischer, alicyclischer, aliphatisch-alicyclischer, heterocyclischer, aliphatisch-heterocyclischer, aromatischer, aliphatisch-aromatischer Kohlenwasserstoffrest mit 1 bis 50 Kohlenstoffatomen, F, Cl, Br, l, -OR²⁵, -COR²⁵, -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, wobei R¹⁷ bis R²⁴ eine gleiche oder unterschiedliche Bedeutung besitzen und kovalent miteinander verknüpft sein können,
R²⁵, R²⁶ = H, substituierter oder unsubstituierter, aliphatischer oder aromatischer Kohlenwasserstoffrest mit 1 bis 25 Kohlenstoffatomen,
M = Alkalimetall-, Erdalkalimetall-, Ammonium-, Phosphoniumion,
wobei die Positionen a und b als Anknüpfpunkte dieses Substituenten im Strukturelement O-Q-O in den Verbindungen der Formeln I, II und III stehen.

Beispiele für W und X sind Kohlenwasserstoffreste, die aliphatisch, alicyclisch, aliphatisch-alicyclisch, heterocyclisch, aliphatisch-heterocylisch, aromatische oder aliphatisch-aromatisch sein können. In den Resten vorhandene Ringsysteme können ihrerseits mit den genannten Kohlenwasserstoffresten substituiert sein. In offenkettigen Strukturelementen können eine oder mehrere Methylengruppen durch Sauerstoff und/oder Schwefel und/oder NR⁷ und/oder NH ersetzt sein und/oder eine oder mehrere CH-Gruppen durch Stickstoff ersetzt sein.

Gegenstand der vorliegenden Erfindung sind auch Bisphosphitmetallkomplexe, enthaltend ein Metall der 4., 5., 6., 7. oder 8. Nebengruppe des Periodensystems der Elemente und ein oder mehrere Bisphosphite der Formeln I, II oder III. Die Substituenten (R¹-R²⁴, Q, X, W) dieser Bisphosphite besitzen die bereits genannten Bedeutungen.

Im Folgenden werden repräsentative Beispiele von Liganden nach den allgemeinen Formeln I, II, III im Sinne dieser Erfindung dargestellt, ohne den Schutzbereich der vorliegenden Erfindung zu beschränken.

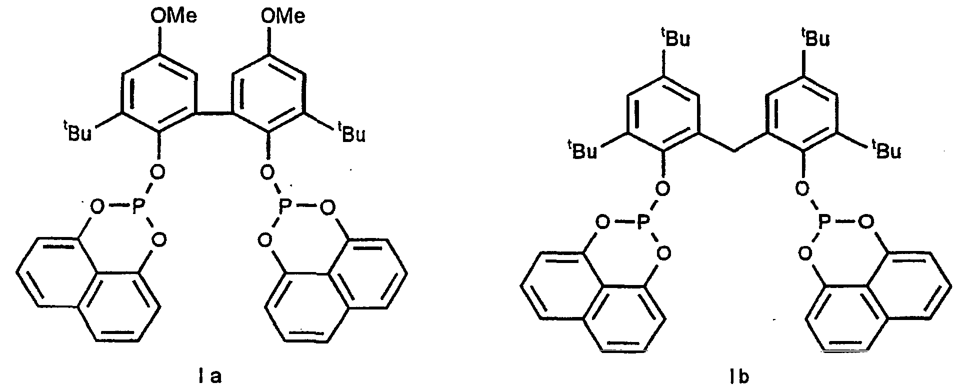

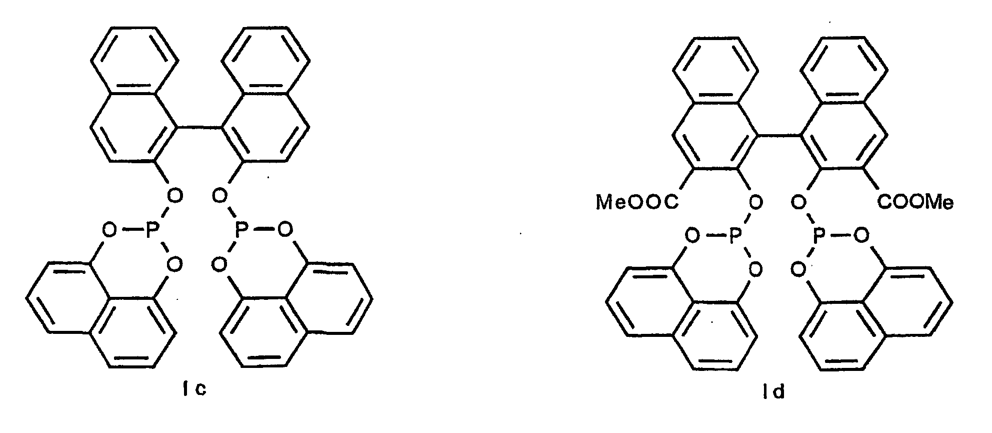

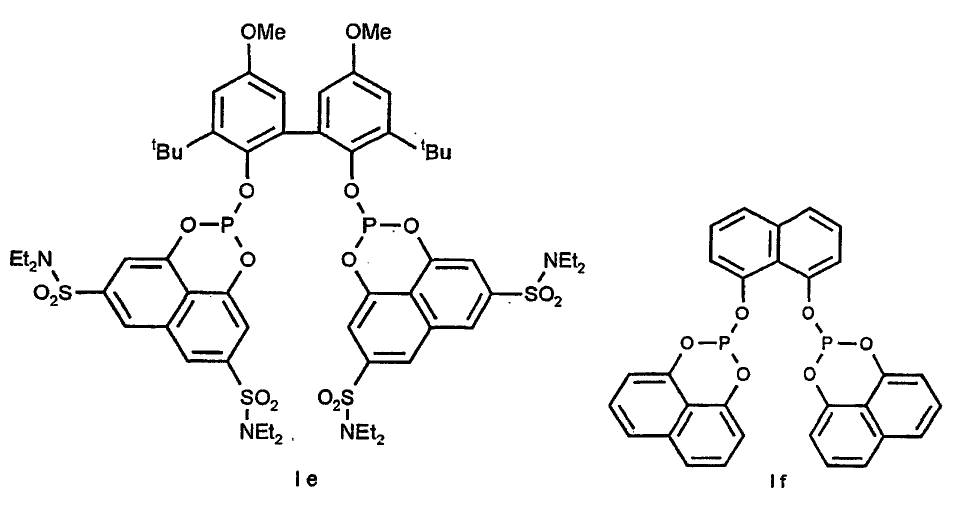

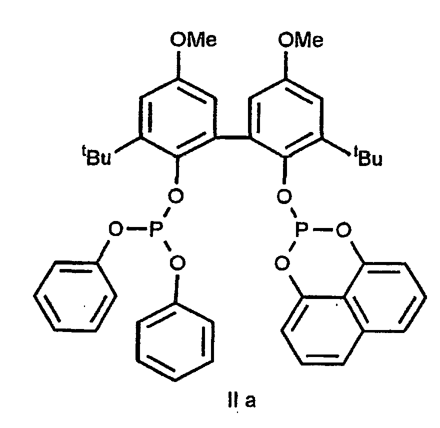

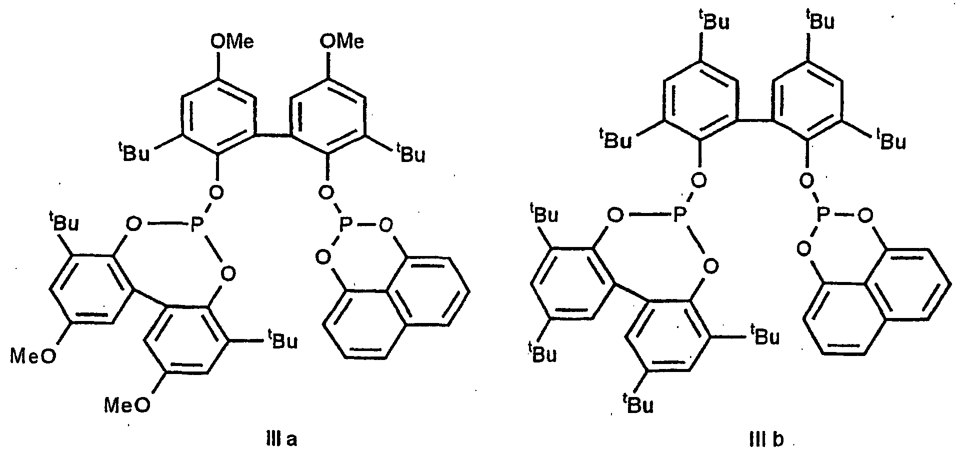

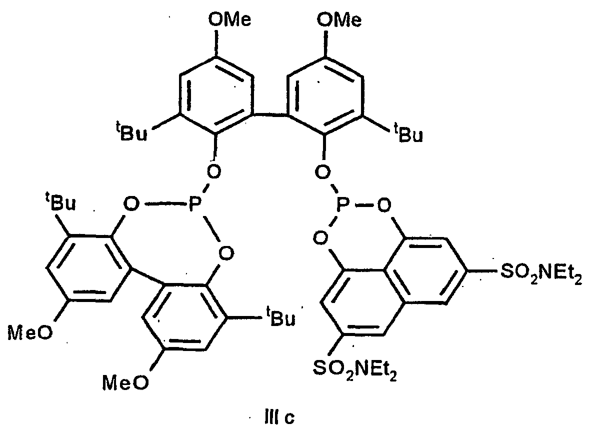

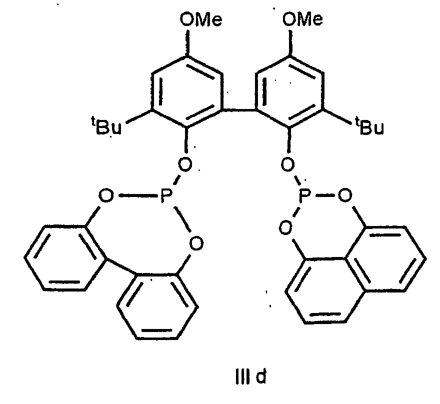

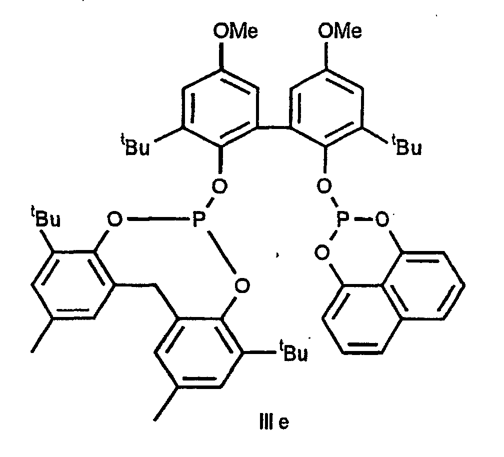

Die erfindungsgemäßen Bisphosphite können durch Reaktionen von Phosphorhalogeniden mit Alkoholen, bei denen Halogenatome gegen Alkoholatgruppen ausgetauscht werden, hergestellt werden:
a) Ein Phosphortrihalogenid, vorzugsweise Phosphortrichlorid, wird mit einem Diol oder zwei Moläquivalenten Alkohol zu einem Monohalogenphosphit (Zwischenprodukt A) umgesetzt.
b) Aus dem Zwischenprodukt A wird durch Reaktion mit einem Diol (HO-Q-OH) ein hydroxyl-substituiertes Phosphit erhalten (Zwischenprodukt B).
c) Ein Phosphortrihalogenid, vorzugsweise Phosphortrichlorid, wird mit ggf. substituierten 1,8-Dihydroxynaphthalin zu einem Monohalogenophosphit umgesetzt **(C).**
d) Aus der Reaktion von Zwischenprodukt **B** mit **C** wird das gewünschte Bisphosphit erhalten.

Symmetrische Bisphosphite nach der allgemeinen Formel III können noch einfacher durch Reaktion der Komponente C mit einem Diol erhalten werden.

Da die eingesetzten Diole und ihre Folgeprodukte häufig fest sind, werden die Umsetzungen im Allgemeinen in Lösungsmitteln durchgeführt. Als Solventien werden nicht protische Lösungsmittel, die weder mit den Diolen noch mit den Phosphorverbindungen reagieren, verwendet. Geeignete Lösungsmittel sind beispielsweise Tetrahydrofuran, Diethylether oder aromatische Kohlenwasserstoffe wie Toluol.

Bei der Umsetzung von Phosphorhalogeniden mit Alkoholen entsteht Halogenwasserstoff, der durch zugegebene Basen gebunden wird. Beispielsweise werden tertiäre Amine, wie Triethylamin, eingesetzt. Es ist auch möglich, die Alkohole vor der Reaktion in Metallalkoholate zu überführen, zum Beispiel durch Reaktion mit Natriumhydrid oder Butyllithium.

Neben diesem Syntheseweg sind noch weitere geeignet, um die erfindungsgemäßen Bisphosphitliganden darzustellen. Dazu zählt zum Beispiel der Einsatz Tris(dialkylamino)phosphinen (als Alternative zum Phosphortrichlorid).

Der 1,8-Dihydroxynaphthalinbaustein, der in den Synthesen eingesetzt wird, kann ebenfalls über verschiedenste Wege erhalten werden. So ist zum Beispiel das 1,8-Dihydroxynaphthalin selbst aus dem 1,8-Naphthalinsulton durch Umsetzung mit Kaliumhydroxid erhältlich (L. Ann. Chem. 1888, 247, 356). Außerdem bieten sich Derivate der Chromotropsäure (4,5-Dihydroxy-2,7-naphthalindisulfonsäure) als Edukte an.

Die erfindungsgemäßen Bisphosphite der Formeln I, II und III sind geeignete Bausteine für die Herstellung von Komplexen mit Metallen der 4., 5., 6., 7. oder 8. Nebengruppe des Periodensystems der Elemente. Insbesondere mit Metallen der 8. Nebengruppe können diese Komplexe als Katalysatoren für Carbonylierungsreaktionen oder Hydroformylierungsreaktion verwendet werden, z. B. für die Hydroformylierung von C2-C25-Olefinen. Die Liganden zeichnen sich durch hohe Hydrolysestabilität aus. Besonders bei Einsatz von Rhodium als Katalysatormetall ergeben sich hohe katalytische Aktivitäten in Hydroformylierungsreaktionen. Aufgrund ihres hohen Molekulargewichtes besitzen die erfindungsgemäßen Bisphosphite eine geringe Flüchtigkeit. Sie können daher einfach von den leichter flüchtigen Reaktionsprodukten abgetrennt werden. Sie sind in den gängigen organischen Solventien ausreichend gut löslich.

Weitere Gegenstände der Erfindung sind die Verwendungen der Bisphosphite bzw. der Bisphosphitmetallkomplexe in Verfahren zur Hydroformylierung von Olefinen, bevorzugt mit 2 bis 25 Kohlenstoffatomen zu den entsprechenden Aldehyden.

Zur Herstellung der katalytisch aktiven Metallkomplexe sind bevorzugt eingesetzte Metalle für die erfindungsgemäßen Bisphosphite Rhodium, Kobalt, Platin und Ruthenium. Aus den erfindungsgemäßen Liganden und dem Metall bildet sich unter Reaktionsbedingungen der aktive Katalysator. Die erfindungsgemäßen Liganden können dabei in freier Form in die Reaktionsmischung gegeben werden. Es ist weiterhin möglich, einen Übergangsmetallkomplex, der die o. g. Bisphosphitliganden enthält, als Precursor für den eigentlichen katalytisch aktiven Komplex einzusetzen. Der Hydroformylierungsprozess kann stöchiometrisch oder mit jeder überschüssigen Menge an freien Bisphosphitliganden durchgeführt werden.

Ferner können auch Mischungen verschiedener Liganden - sowohl der erfindungsgemäßen Bisphosphite als auch anderer geeigneter Phosphorhaltiger Liganden - als freie Ligandkomponente vorhanden sein.

Als zusätzliche, im Reaktionsgemisch vorhandene Liganden können Phosphine, Phosphite, Phosphonite oder Phosphinite eingesetzt werden.

### Beispiele für solche Liganden sind:

Phosphine: Triphenylphosphin, Tris(p-tolyl)phosphin, Tris(m-tolyl)phosphin, Tris(o-tolyl)phosphin, Tris(p-methoxyphenyl)phosphin, Tris(p-dimethylaminophenyl)-phosphin, Tricyclohexylphosphin, Tricyclopentylphosphin, Triethylphosphin, Tri-(1-naphthyl)phosphin, Tribenzylphosphin, Tri-n-butylphosphin, Tri-t-butylphosphin. Phosphite: Trimethylphosphit, Triethylphosphit, Tri-n-propylphosphit, Tri-ipropylphosphit, Tri-n-butylphosphit, Tri-i-butylphosphit, Tri-t-butylphosphit, Tris(2-ethylhexyl)phosphit, Triphenylphosphit, Tris(2.4-di-t-butylphenyl)phosphit, Tris(2-tbutyl-4-methoxyphenyl)phosphit, Tris(2-t-butyl-4-methylphenyl)phosphit, Tris(pkresyl)phosphit. Außerdem sind sterisch gehinderte Phosphitliganden, wie sie unter anderem in EP 155 508, US 4 668 651, US 4 748 261, US 4 769 498, US 4 774 361, US 4 835 299, US 4 885 401, US 5 059 710, US 5113 022, US 5 179 055, US 5 260 491, US 5 264 616, US 5 288 918, US 5 360 938, EP 472 071, EP 518 241 und WO 97/20795 beschriebenen werden, geeignete Liganden.

Phosphonite: Methyldiethoxyphosphin, Phenyldimethoxyphosphin, Phenyldiphenoxyphosphin, 6-Phenoxy-6H-dibenz[c,e][1,2]oxaphosphorin und dessen Derivate, in denen die Wasserstoffatome ganz oder teilweise durch Alkyl-, Arylreste oder Halogenatome ersetzt sind und Liganden, die in WO 98 43935, JP 09-268152 und DE 198 10 794 und in den deutschen Patentanmeldungen DE 199 54 721 und DE 199 54 510 beschrieben sind.

Gängige Phosphinitliganden sind unter anderem in US 5 710 344, WO 95 06627, US 5 360 938 oder JP 07082281 beschrieben. Beispiele hierfür sind Diphenyl(phenoxy)-phosphin und dessen Derivate, in denen die Wasserstoffatome ganz oder teilweise durch Alkyl-, Arylreste oder Halogenatome ersetzt sind, Diphenyl(methoxy)phosphin, Diphenyl(ethoxy)phosphin usw.

Im Allgemeinen werden 1 bis 500, vorzugsweise 1 bis 200, bevorzugt 3 bis 50 Mol des erfindungsgemäßen Liganden pro Mol Gruppe-VIII-Übergangsmetall eingesetzt. Frischer Ligand kann zu jedem Zeitprodukt der Reaktion zugesetzt werden, um die Konzentration an freiem Liganden konstant zu halten. Die erfindungsgemäßen Übergangsmetall-Bisphosphitkomplex-Katalysatoren können vor ihrem Einsatz synthetisiert werden. In der Regel werden aber die katalytisch aktiven Komplexe aus einem Katalysatorvorläufer und dem erfindungsgemäßen Bisphosphitliganden in situ im Reaktionsmedium gebildet.

Als Katalysatorvorläufer kommen Salze oder Komplexe der Übergangsmetalle zum Einsatz. Beispiele sind Rhodiumcarbonyle, Rhodiumnitrat, Rhodiumchlorid, Rh(CO)₂(acac) (acac = Acetylacetonat), Rhodiumacetat, Rhodiumoctanoat oder Rhodiumnonanoat.

Die Konzentration des Metalls im Reaktionsgemisch liegt im Bereich von 1 ppm bis 1000 ppm, vorzugsweise im Bereich von 5 ppm bis 300 ppm.

Die mit den erfindungsgemäßen Bisphosphiten bzw. den entsprechenden Metallkomplexen durchgeführte Hydroformylierungsreaktion erfolgt nach bekannten Vorschriften, wie z. B. in J. FALBE, "New Syntheses with Carbon Monoxide", Springer Verlag, Berlin, Heidelberg, New York, Seite 95 ff., (1980) beschrieben.

Die Reaktionstemperaturen für ein Hydroformylierungsverfahren mit den erfindungsgemäßen Bisphosphiten bzw. Bisphosphitmetallkomplexen als Katalysator liegen zwischen 40 °C und 180 °C, vorzugsweise zwischen 75 °C und 140 °C. Die Drücke, unter denen die Hydroformylierung abläuft, betragen 1-300 bar Synthesegas, vorzugsweise 15-64 bar. Das Molverhältnis zwischen Wasserstoff und Kohlenmonoxid (H₂/CO) im Synthesegas beträgt 10/1 bis 1/10, bevorzugt 1/1 bis 211.

Der Katalysator bzw. der Ligand ist homogen im Hydroformylierungsgemisch, bestehend aus Edukt (Olefine) und Produkten (Aldehyden, Alkoholen, im Prozess gebildete Hochsieder), gelöst. Optional kann zusätzlich ein Lösungsmittel verwendet werden.

Die Edukte für die Hydroformylierung sind Monoolefine oder Gemische von Monoolefinen mit 2 bis 25 Kohlenstoffatomen mit end- oder innenständiger C-C-Doppelbindung. Sie können geradkettig, verzweigt oder von cyclischer Struktur sein und auch mehrere olefinisch ungesättigte Gruppen aufweisen. Beispiele sind Propen, 1-Buten, c-2-Buten, t-2-Buten, Isobuten, Butadien, Mischungen der C4-Olefine, 1-oder 2-Penten, 2-Methylbuten-1, 2-Methylbuten-2, 3-Methylbuten-1, 1-, 2- oder 3-Hexen, das bei der Dimerisierung von Propen anfallende C6-Olefingemisch (Dipropen), 1-Hepten, Heptene, 2- oder 3-Methyl-1-hexen, 1-Octen, Octene, 2-Methylheptene, 3-Methylheptene, 5-Methylhepten-2, 6-Methylhepten-2, 2-Ethylhexen-1, das bei der Dimerisierung von Butenen anfallende isomere C8-Olefingemisch (Dibuten), 1-Nonen, Nonene, 2- oder 3-Methyloctene, das bei der Trimerisierung von Propen anfallende C9-Olefingemisch (Tripropen), Decene, 2-Ethyl-1-octen, Dodecene, das bei der Tetramerisierung von Propen oder der Trimerisierung von Butenen anfallende C12-Olefingemisch (Tetrapropen oder Tributen), Tetradecene, Hexadecene, bei der Tetramerisierung von Butenen anfallende C16-Olefingemisch (Tetrabuten) sowie durch Cooligomerisierung von Olefinen mit unterschiedlicher C-Zahl (bevorzugt 2 bis 4) hergestellte Olefingemische, gegebenenfalls nach destillativer Trennung in Fraktionen mit gleicher oder ähnlicher C-Zahl. Ebenfalls können Olefine oder Olefingemische, die durch Fischer-Tropsch-Synthese erzeugt werden, eingesetzt werden, sowie Olefine, die durch Oligomerisierung von Ethen erhalten werden oder die über Methathesereaktionen oder Telomerisationsreaktion zugänglich sind.

Bevorzugte Edukte sind Propen, 1-Buten, 2-Buten, 1-Hexen, 1-Octen, Dimere und Trimere des Butens (Dibuten, Di-n-buten, Di-iso-buten, Tributen) und allgemein α-Olefine.

Die Hydroformierung kann kontinuierlich oder diskontinuierlich durchgeführt werden. Beispiele für technische Ausführungen sind Rührkessel, Blasensäulen, Strahldüsenreaktoren, Rohrreaktoren, oder Schlaufenreaktoren, die zum Teil kaskadiert und/oder mit Einbauten versehen sein können.

Die Reaktion kann durchgehend oder in mehreren Stufen erfolgen. Die Trennung der entstandenen Aldehydverbindungen und des Katalysators kann durch eine herkömmliche Methode, wie Fraktionierung, durchgeführt werden. Technisch kann dies beispielsweise über eine Destillation, über einen Fallfilmverdampfer oder einen Dünnschichtverdampfer erfolgen. Die gilt besonders, wenn der Katalysator in einem hochsiedenden Lösungsmittel gelöst von den niedriger siedenden Produkten abgetrennt wird. Die abgetrennte Katalysatorlösung kann für eine weitere Hydroformylierungen verwendet werden. Bei Einsatz niederer Olefine (z. B. Propen, Buten, Penten) ist auch ein Austrag der Produkte aus dem Reaktor über die Gasphase möglich.

Die folgenden Beispiele sollen die Erfindung erläutern, nicht aber ihren Anwendungsbereich beschränken, der sich aus den Patentansprüchen ergibt.

### Beispiele

Alle Präparationen wurden mit Standard-Schlenk-Technik unter Schutzgas durchgeführt. Die Lösungsmittel wurden vor Gebrauch über geeigneten Trocknungsmitteln getrocknet.

### Beispiel 1

### Synthese des Liganden III a

Das als Vorstufe eingesetzte Hydroxyphosphit (CAN 108609-96-7) wurde nach US 4 885 401 synthetisiert. 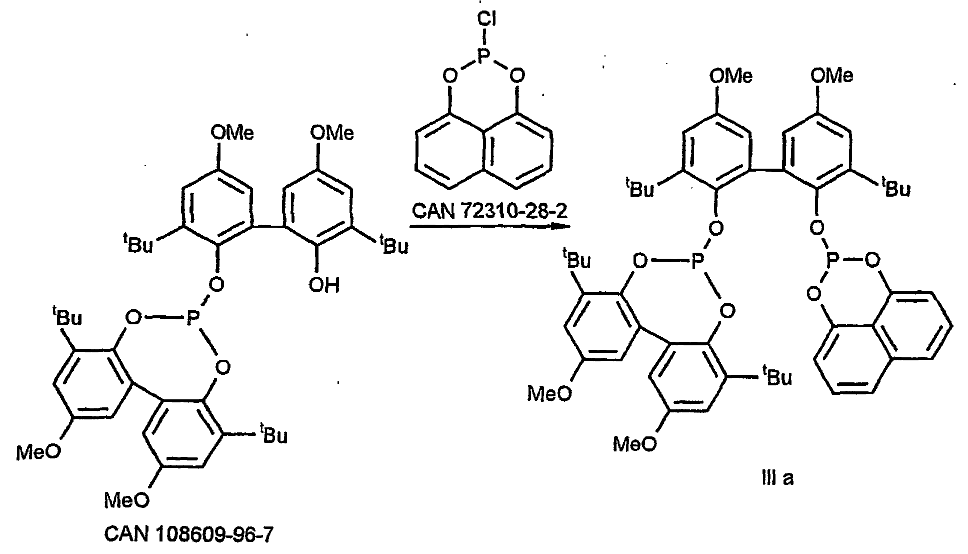 25,69 g (34,5 mmol) des Phosphits (CAN 108609-96-7) und 8,1 g Triethylamin werden unter Rühren in 100 ml Toluol gelöst. Diese Lösung tropft man langsam bei -40 °C in eine Lösung von 8,0 g (35,6 mmol) des Chlorophosphits (CAN 72310-28-2) in 100 ml Toluol. Nach beendeter Zugabe lässt man auf Raumtemperatur erwärmen und erhitzt dann die Mischung für 3 Stunden auf 60 °C. Nach dem Abkühlen auf Raumtemperatur wird der ausgefallene Feststoff abfiltriert und verworfen. Das Lösungsmittel wird im Vakuum entfernt, der Rückstand mit 200 ml Pentan gerührt. Der hierbei anfallende Feststoff wird isoliert, mit Acetonitril gewaschen und im Vakuum getrocknet. Ausbeute 20,2 g (63 %).
¹H NMR (C₇D₈) δ = 7,2-6,2 (14 H, Ar-H), 3,5-3,0 (12 H, OMe), 1,4-0,9 (36 H, ^{t}Bu) ³¹P{¹H} NMR (C₇D₈) δ =134, 108 ppm

### Beispiel 2

### Synthese des Liganden Ia

9,50 g (26,5 mmol) 3,3'-Di-t-butyl-2,2'-dihydroxy-5,5'-dihydroxybiphenyl werden zusammen mit 12,50 g Triethylamin in 110 ml Toluol gelöst. Zu dieser Lösung gibt man innerhalb von einer Stunde 11,96 g (53,3 mmol) der Chlorophosphitkomponente (CAN 72310-28-2), gelöst in 100 ml Toluol. Nach beendeter Zugabe wird die Lösung noch 3 Stunden gerührt, das ausgefallene Triethylammoniumchlorid abfiltriert und die Lösung zur Trockne eingeengt. Nach Umkristallisation aus Acetonitril erhält man das Bisphosphit I a (49 % Ausbeute).
¹H NMR (C₇D₈) δ = 7,2-6,4 (16 H, Ar-H), 3,9-3,7 (6 H, OMe), 1,15 (18 H, ^{t}Bu) ³¹P NMR (C₇D₈) δ = 105,9 ppm

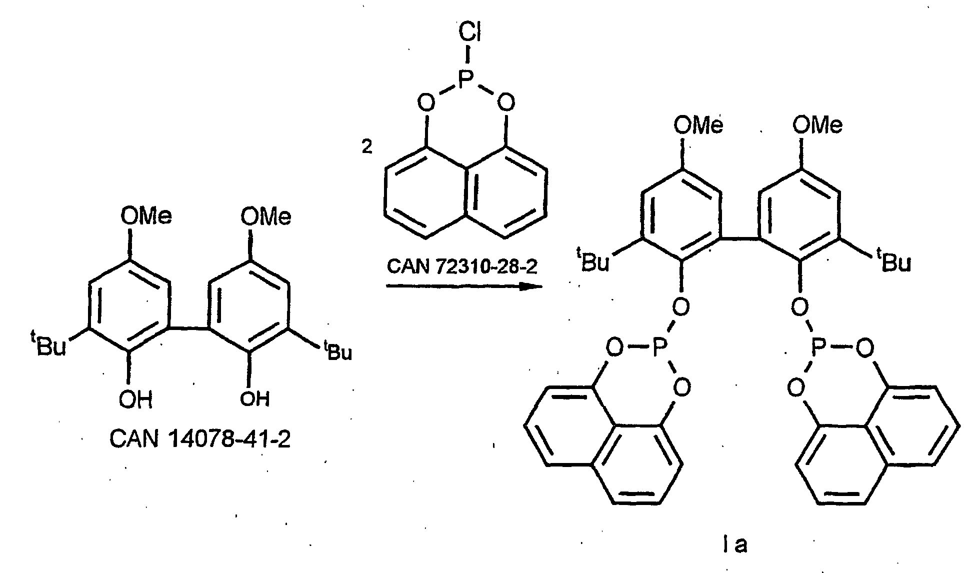

### Beispiel 3

### Hydroformylierung von 1-Octen

Die Versuche wurden in einem 300 ml Laborautoklaven (Fa. Berghof) durchgeführt, ausgestattet mit Innenthermometer und einer Kapillare zur Entnahme von Proben während der Reaktion. Olefin und ein Teil des Lösungsmittels werden im Autoklav vorgelegt, der Katalysator, bestehend aus Rh-Precursor und Ligand, gelöst im Rest des Lösungsmittels, wird zum Start der Reaktion aus einer Druckbürette zugegeben. Als Rh-Precursor wird in allen Autoklavenversuchen Rhodiumnonanoat eingesetzt.

In einem Autoklav dieses Typs wurden 60 g 1-Octen in 100 g Toluol hydroformyliert. Die Ergebnisse sind in der folgenden Tabelle zusammengefasst.

| Beispiel Nr. | B3-1 | B3-2 | B3-3 | B3-4 |
|---|---|---|---|---|
| Ligand No. | **3.a** | **3.a** | **3.a** | **3.a** |
| Temperatur [°C] | 100 | 100 | 100 | 100 |
| L/Rh[mol/mol] | 5 | 4 | 5 | 8 |
| Synthesegasdruck [bar] | 20 | 20 | 50 | 20 |
| Konzentration Rh [ppm] | 41 | 41 | 43 | 44 |
| Umsatz Olefin | 91.5 | 92.3 | 94.7 | 93.2 |

| Analyse Aldehyde | | | | |
|---|---|---|---|---|
| Nonanal | 81.9 | 85.1 | 81.5 | 84.4 |
| 2-Methyloctanal | 15.8 | 13.5 | 17.2 | 13.8 |
| 3-Ethylheptanal | 2.0 | 1.3 | 1.3 | 1.5 |
| 4-Propylhexanal | 0.3 | 0.1 | 0.0 | 0.3 |

### Beispiel 4

### Hydroformylierung einer Mischung von Octenen

In dem in Beispiel 3 beschriebenen Autoklav wurden 60 g einer Mischung aus Octenen (1-Octen 3,1 %, 2-Octen 49,0 %, 3-Octen 33,0 %, 4-Octen 14,9 %), gelöst in 100 g Toluol, hydroformyliert. Die Reaktion wurde über Probenahmen verfolgt, die Ergebnisse nach 8 Stunden Reaktionszeit sind in der folgenden Tabelle zusammengefasst.

| Beispiel Nr. | B4-1 | B4-2 |
|---|---|---|
| Ligand No. | **3.a** | **3.a** |
| Temperatur [°C] | 130 | 130 |
| URh [mol/mol] | 5 | 5 |
| Synthesegasdruck [bar] | 20 | 50 |
| Konzentration Rh [ppm] | 100 | 100 |
| Umsatz Olefin | 97.1% | 98.0 |

| Analyse Aldehyde | | |
|---|---|---|
| Nonanal | 57.6 | 36.8 |
| 2-Methyloctanal | 27.3 | 36.2 |
| 3-Ethylheptana | 8.0 | 13.6 |
| 4-Propylhexanal | 7.1 | 13.4 |

### Beispiel 5

### Hydroformylierung von Propen

In dem in Beispiel 3 beschriebenen Autoklav wurden 30 g Propen, gelöst in 150 g Toluol, hydroformyliert. Die Reaktion wurde über Probenahmen verfolgt, die Ergebnisse nach 5 Stunden Reaktionszeit sind in der folgenden Tabelle zusammengefasst.

| Beispiel Nr. | B5-1 |
|---|---|
| Ligand No. | **3.a** |
| Temperatur [°C] | 80 |
| L/Rh [mol/mol] | 2.5 |
| Synthesegasdruck [bar] | 20 |
| Konzentration Rh [ppm] | 41 |
| Umsatz Olefin | 98% |

| Analyse Aldehyde | |
|---|---|
| Butanal | 70.5 |
| 2-Metylpropanal | 29.5 |

### Beispiel 6

### Hydroformylierung von Buten

In dem in Beispiel 3 beschriebenen Autoklav wurden 10 g Buten, gelöst in 100 g Toluol, hydroformyliert. Die Reaktion wurde über Probenahmen verfolgt, die Ergebnisse nach 5 Stunden Reaktionszeit sind in der folgenden Tabelle zusammengefasst.

| Beispiel Nr. | B6-1 | B6-2 | B6-3 |
|---|---|---|---|
| Ligand No. | **3.a** | **3.a** | **3.b** |
| Olefin | 1-Buten | t2-Buten | c2-Buten |
| Temperatur [°C] | 80 | 80 | 80 |
| L/Rh[mol/mol] | 2.5 | 2.5 | 2.5 |
| Synthesegasdruck [bar] | 20 | 20 | 20 |
| Konzentration Rh [ppm] | 60 | 60 | 60 |
| Umsatz Olefin | 95.3% | 44.4% | 68.0% |

| Analyse Aldehyde | | | |
|---|---|---|---|
| Pentanal | 83.8 | 24.6 | 25.8 |
| 2-Metylbutanal | 16.2 | 75.4 | 74.2 |

## Patentansprüche

1. Bisphosphit der Formel I 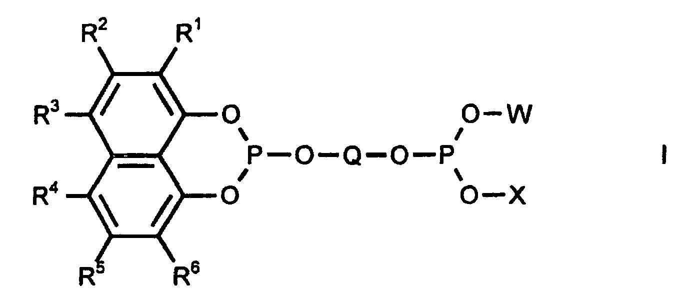 mit
R¹, R², R³, R⁴, R⁵, R⁶ = H, aliphatischer, alicyclischer, aliphatisch-alicyclischer, heterocyclischer, aliphatisch-heterocyclischer, aromatischer, aliphatisch-aromatischer Kohlenwasserstoffrest mit 1 bis 50 Kohlenstoffatomen, F, Cl, Br, I, -OR⁷, -COR⁷, -CO₂R⁷, -CO₂M, -SR⁷, -SO₂R⁷, -SOR⁷, -SO₃R⁷, -SO₃M, -SO₂NR⁷R⁸, NR⁷R⁸, N=CR⁷R⁸, NH₂, wobei R¹ bis R⁶ eine gleiche oder unterschiedliche Bedeutung besitzen und kovalent miteinander verknüpft sein können,
R⁷, R⁸ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion,
Q = ein Kohlenwasserstoffrest gemäß Formel IV 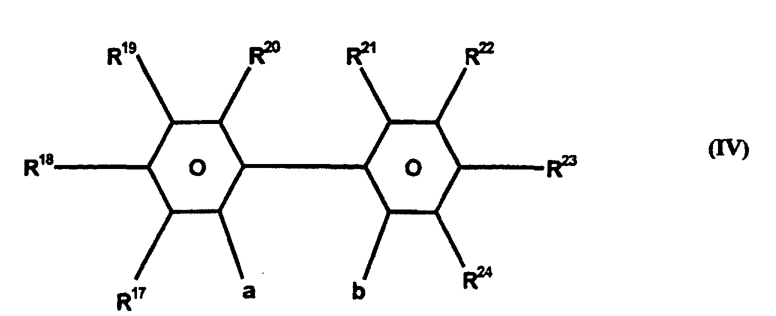 mit
R¹⁷, R¹⁸, R¹⁹, R²⁰, R²¹, R²², R²³, R²⁴ = H, aliphatischer, alicyclischer, aliphatisch-alicyclischer, heterocyclischer, aliphatisch-heterocyclischer, aromatischer, aliphatisch-aromatischer Kohlenwasserstoffrest mit 1 bis 50 Kohlenstoffatomen, F, Cl, Br, I, -OR²⁵, -COR²⁵, -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, wobei R¹ bis R⁶ eine gleiche oder unterschiedliche Bedeutung besitzen und kovalent miteinander verknüpft sein können,
R²⁵, R²⁶ = H, substituierter oder unsubstituierter, aliphatischer oder aromatischer Kohlenwasserstoffrest mit 1 bis 25 Kohlenstoffatomen,
M = Alkalimetall-, Erdalkalimetall-, Ammonium-, Phosphoniumion,
wobei die Positionen a und b als Anknüpfpunkte dienen,
W, X = aliphatische, alicyclische, aliphatisch-alicyclische, heterocyclische, aliphatisch-heterocyclische, aromatische, aliphatisch-aromatische Kohlenwasserstoffreste mit 1 bis 50 kohlenstoffatomen, die gleich oder unterschiedlich oder kovalent miteinander verknüpft sein können.

2. Bisphosphit gemäß Anspruch 1,
**dadurch gekennzeichnet,**
**dass** W und X aliphatische, alicyclische, aliphatisch-alicyclische, heterocyclische, aliphatisch-heterocyclische, aromatische, aliphatisch-aromatische Kohlenwasserstoffreste mit 1 bis 50 Kohlenstoffatomen, mit einer kovalenten Verknüpfung gemäß Formel II 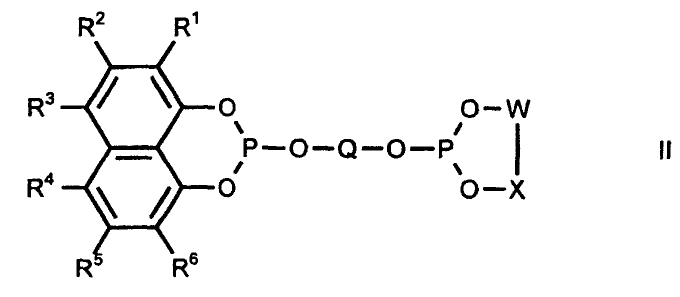 sind und R¹, R², R³, R⁴, R⁵, R⁶ und Q die in Anspruch 1 genannten Bedeutungen besitzen.

3. Bisphosphit gemäß Anspruch 1,
**dadurch gekennzeichnet,**
**dass** W und X aromatische Kohlenwasserstoffreste mit 1 bis 50 Kohlenstoffatomen mit kovalenten Verknüpfungen gemäß Formel III 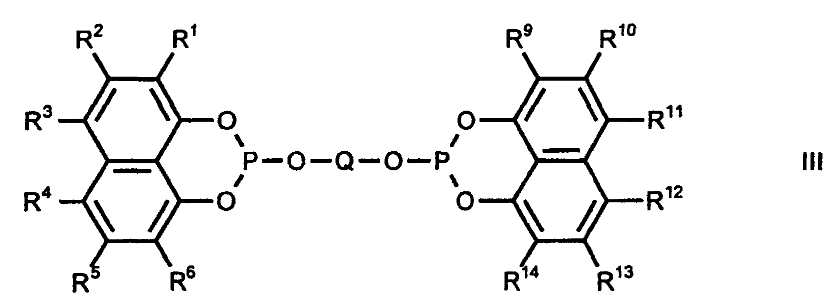 sind,
mit R⁹, R¹⁰, R¹¹, R¹², R¹³, R¹⁴ = H, aliphatischer, alicyclischer, aliphatisch-alicyclischer, heterocyclischer, aliphatisch-heterocyclischer, aromatischer, aliphatisch-aromatischer Kohlenwasserstoffrest mit 1 bis 50 Kohlenstoffatomen, F, Cl, Br, l, -OR¹⁵, -COR¹⁵, -CO₂R¹⁵, -CO₂M, -SR¹⁵, -SO₂R¹⁵, -SOR¹⁵, -SO₃R¹⁵, -SO₃M, -SO₂NR¹⁵R¹⁶, NR¹⁵R¹⁶, N=CR¹⁵R¹⁶, NH₂, wobei R⁹ bis R¹⁴ eine gleiche oder unterschiedliche Bedeutung besitzen und kovalent miteinander verknüpft sein können,
R¹⁵, R¹⁶ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion und
R¹, R², R³, R⁴, R⁵, R⁶ und Q die in Anspruch 1 genannten Bedeutungen besitzen.

4. Bisphosphitmetallkomplex, enthaltend ein Metall der 4., 5., 6., 7. oder 8. Nebengruppe des Periodensystems der Elemente und ein oder mehrere Bisphosphite der Formel I 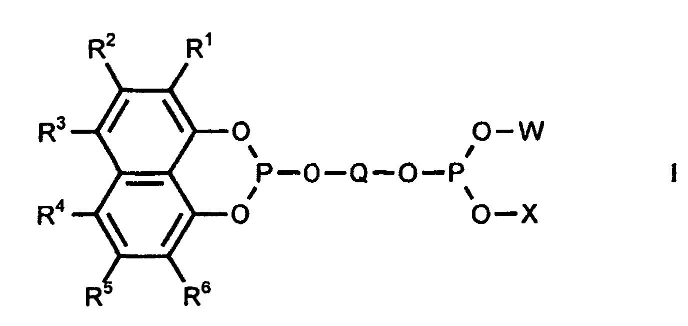 mit
R¹, R², R³, R⁴, R⁵, R⁶ = H, aliphatischer, alicyclischer, aliphatisch-alicyclischer, heterocyclischer, aliphatisch-heterocyclischer, aromatischer, aliphatisch-aromatischer Kohlenwasserstoffrest mit 1 bis 50 Kohlenstoffatomen, F, Cl, Br, I, -OR⁷, -COR⁷, -CO₂R⁷, -CO₂M, -SR⁷, -SO₂R⁷, -SOR⁷, -SO₃R⁷, -SO₃M, -SO₂NR⁷R⁸, NR⁷R⁸, N=CR⁷R⁸, NH₂, wobei R¹ bis R¹ R⁶ eine gleiche oder unterschiedliche Bedeutung besitzen und kovalent miteinander verknüpft sein können,
R⁷, R⁸ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion,
Q = ein Kohlenwasserstoffrest gemäß Formel IV 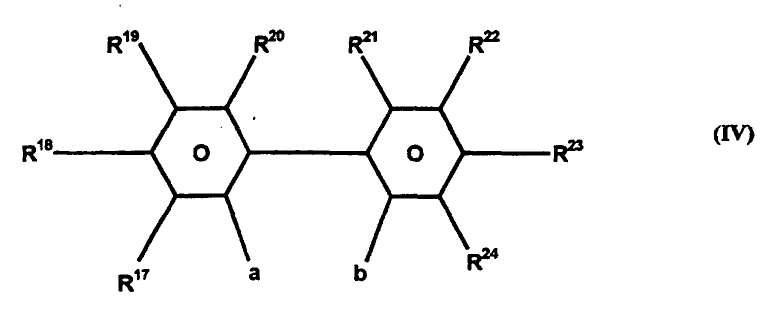 mit
R¹⁷, R¹⁸, R¹⁹, R²⁰, R²¹, R²², R²³, R²⁴ = H, aliphatischer, alicyclischer, aliphatisch-alicyclischer, heterocyclischer, aliphatisch-heterocyclischer, aromatischer, aliphatisch-aromatischer Kohlenwasserstoffrest mit 1 bis 50 Kohlenstoffatomen, F, Cl, Br, l, -OR²⁵, -COR²⁵, -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, wobei R¹⁷ bis R²⁴ eine gleiche oder unterschiedliche Bedeutung besitzen und kovalent miteinander verknüpft sein können,
R²⁵, R²⁶ = H, substituierter oder unsubstituierter, aliphatischer oder aromatischer Kohlenwasserstoffrest mit 1 bis 25 Kohlenstoffatomen,
M = Alkalimetall-, Erdalkalimetall-, Ammonium-, Phosphoniumion
wobei die Positionen a und b als Anknüpfpunkte dienen,
W, X = aliphatische, alicyclische, aliphatisch-alicyclische, heterocyclische, aliphatisch-heterocyclische, aromatische, aliphatisch-aromatische Kohlenwasserstoffreste mit 1 bis 50 Kohlenstoffatomen, die gleich oder unterschiedlich oder kovalent miteinander verknüpft sein können.

5. Bisphosphitmetallkomplex nach Anspruche 4,
**dadurch gekennzeichnet,**
**dass** W und X aliphatische, alicyclische, aliphatisch-alicyclische, heterocyclische, aliphatisch-heterocyclische, aromatische, aliphatisch-aromatische Kohlenwasserstoffreste mit 1 bis 50 Kohlenstoffatomen, mit einer kovalenten Verknüpfung gemäß Formel II 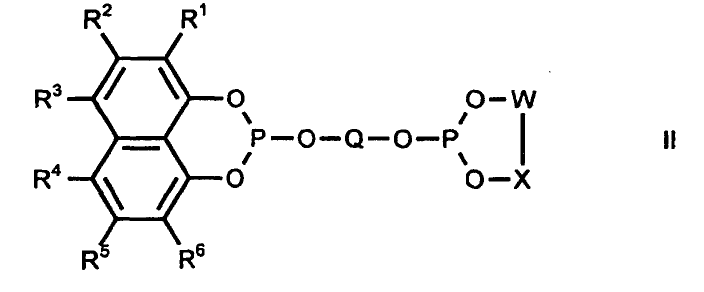 sind und R¹, R², R³, R⁴, R⁵, R⁶ und Q die in Anspruch 1 genannten Bedeutungen besitzen.

6. Bisphosphitmetallkomplex nach Anspruch 4,
**dadurch gekennzeichnet,**
**dass** W und X aromatische Kohlenwasserstoffreste mit 1 bis 50 Kohlenstoffatomen mit kovalenten Verknüpfungen gemäß Formel III 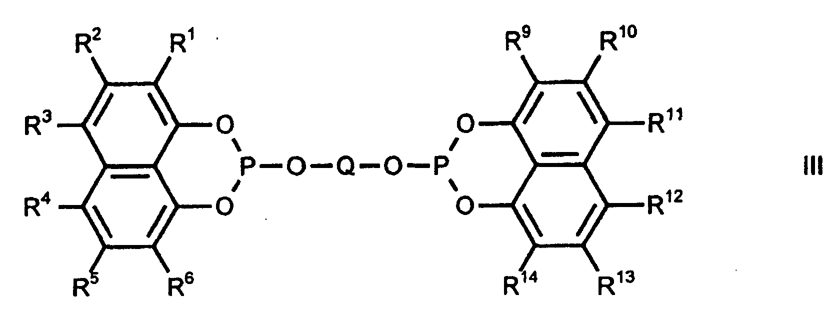 sind,
mit R⁹, R¹⁰, R¹¹, R¹², R¹³, R¹⁴ = H, aliphatischer, alicyclischer, aliphatisch-alicyclischer, heterocyclischer, aliphatisch-heterocyclischer, aromatischer, aliphatisch-aromatischer Kohlenwasserstoffrest mit 1 bis 50 Kohlenstoffatomen, F, Cl, Br, l, -OR¹⁵, -COR¹⁵, -CO₂R¹⁵, -CO₂M, -SR¹⁵, -SO₂R¹⁵, -SOR¹⁵, -SO₃R¹⁵, -SO₃M, -SO₂NR¹⁵R¹⁶, NR¹⁵R¹⁶, N=CR¹⁵R¹⁶, NH₂, wobei R⁹ bis R¹⁴ eine gleiche oder unterschiedliche Bedeutung besitzen und kovalent miteinander verknüpft sein können,
R¹⁵, R¹⁶ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion und
R¹, R², R³, R⁴, R⁵, R⁶ und Q die in Anspruch 1 genannten Bedeutungen besitzen.

7. Bisphosphitmetalikomplex nach einem der Ansprüche 4 bis 6,
**dadurch gekennzeichnet,**
**dass** als Metall Rhodium, Platin, Kobalt oder Ruthenium eingesetzt wird.

8. Verwendung der Bisphosphite gemäß einem der Ansprüche 1 bis 3 in einem Verfahren zur Hydroformylierung von Olefinen.

9. Verwendung der Bisphosphitmetallkomplexe gemäß einem der Ansprüche 4 bis 7 in einem Verfahren zur Hydroformylierung von Olefinen.

10. Verwendung der Bisphosphite gemäß einem der Ansprüche 1 bis 3 in einem Verfahren zur Hydroformylierung von Olefinen unter Anwesenheit von weiteren phosphorhaltigen Liganden.

11. Verwendung der Bisphosphitmetallkomplexe gemäß einem der Ansprüche 4 bis 7 in einem Verfahren zur Hydroformylierung von Olefinen unter Anwesenheit von weiteren phosphorhaltigen Liganden.

## Claims

1. A bisphosphite of the formula I 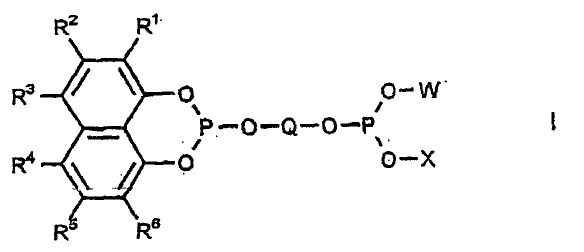 where
R¹, R², R³, R⁴, R⁵, R⁶ = H, an aliphatic, alicyclic, aliphatic-alicyclic, heterocyclic, aliphatic-heterocyclic, aromatic or aliphatic-aromatic hydrocarbon radical having from 1 to 50 carbon atoms, F, Cl, Br, I, -OR⁷, -COR⁷, -CO₂R⁷, -CO₂M, -SR⁷, -SO₂R⁷, -SOR⁷, -SO₃R⁷, -SO₃M, -SO₂NR⁷R⁸, NR⁷R⁸, N=CR⁷R⁸, NH₂, where R¹ to R⁶ are identical or different and may be covalently linked to one another,
R⁷, R⁸ = H or a substituted or unsubstituted, aliphatic or aromatic hydrocarbon radical having from 1 to 25 carbon atoms, where R⁷ and R⁸ are identical or different,
M = an alkali metal ion, alkaline earth metal ion, ammonium ion or phosphonium ion,
Q = a hydrocarbon radical of the formula IV 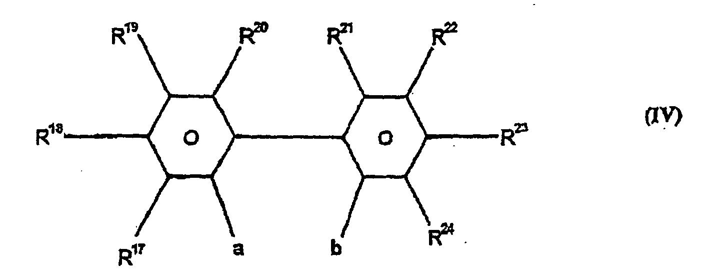 where
R¹⁷, R¹⁸, R¹⁹, R²⁰, R²¹, R²², R²³, R²⁴ = H, an aliphatic, alicyclic, aliphatic-alicyclic, heterocyclic, aliphatic-heterocyclic, aromatic or aliphatic-aromatic hydrocarbon radical having from 1 to 50 carbon atoms, F, Cl Br, I, -OR²⁵, -COR²⁵ -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂,
where R¹ to R⁶ are identical or different and may be covalently linked to one another,
R²⁵, R²⁶ = H or a substituted or unsubstituted, aliphatic or aromatic hydrocarbon radical having from 1 to 25 carbon atoms,
M = an alkali metal ion, alkaline earth metal ion, ammonium ion or phosphonium ion,
where the positions a and b serve as linkage points,
W, X = aliphatic, alicyclic, aliphatic-alicyclic, heterocyclic, aliphatic-heterocyclic, aromatic or aliphatic-aromatic hydrocarbon radicals having from 1 to 50 carbon atoms, which may be identical or different or may be covalently linked to one another.

2. A bisphosphite according to claim 1, **characterized in that**
W and X are aliphatic, alicyclic, aliphatic-alicyclic, heterocyclic, aliphatic-heterocyclic, aromatic or aliphatic-aromatic hydrocarbon radicals which have from 1 to 50 carbon atoms and are covalently linked as in the formula II 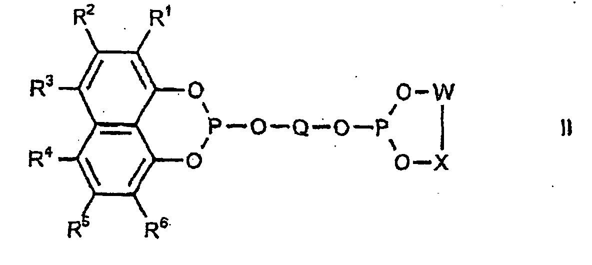 and R¹, R², R³, R⁴, R⁵, R⁶ and Q are as defined in claim 1.

3. A bisphosphite according to claim 1, **characterized in that**
W and X are aromatic hydrocarbon radicals which have from 1 to 50 carbon atoms and are covalently linked as in the formula III 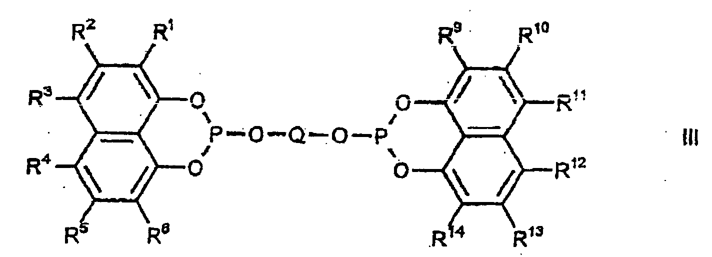 where R⁹, R¹⁰, R¹¹, R¹², R¹³, R¹⁴ = H, an aliphatic, alicyclic, aliphatic-alicyclic, heterocyclic, aliphatic-heterocyclic, aromatic or aliphatic-aromatic hydrocarbon radical having from 1 to 50 carbon atoms, F, Cl, Br, I, -OR¹⁵, -COR¹⁵, -CO₂R¹⁵, -CO₂M, -SR¹⁵, -SO₂R¹⁵, -SOR¹⁵, -SO₃R¹⁵, -SO₃M, -SO₂NR¹⁵R¹⁶, NR¹⁵R¹⁶, N=CR¹⁵R¹⁶, NH₂, where R⁹ to R¹⁴ are identical or different and may be covalently linked to one another,
R¹⁵, R¹⁶ = H or a substituted or unsubstituted, aliphatic or aromatic hydrocarbon radical having from 1 to 25 carbon atoms, where R¹⁵ and R¹⁶ : are identical or different,
M = an alkali metal ion, alkaline earth metal ion, ammonium ion or phosphonium ion and
R¹, R², R³, R⁴, R⁵, R⁶ and Q are as defined in claim 1.

4. A bisphosphite-metal complex comprising a metal of transition group 4, 5, 6, 7 or 8 of the Periodic Table of the Elements and one or more bisphosphites of the formula I 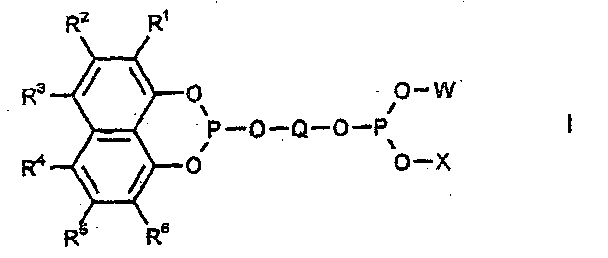 where
R¹, R², R³, R⁴, R⁵, R⁶ = H, an aliphatic, alicyclic, aliphatic-alicyclic, heterocyclic, aliphatic-heterocyclic, aromatic or aliphatic-aromatic hydrocarbon radical having from 1 to 50 carbon atoms, F, Cl, Br, I, -OR⁷, -COR⁷, -CO₂R⁷, -CO₂M, -SR⁷, -SO₂R⁷, -SOR⁷, -SO₃R⁷, -SO₃M, -SO₂NR⁷R⁸, NR⁷R⁸, N=CR⁷R⁸, NH₂, where R¹ to R₆ are identical or different and may be covalently linked to one another,
R⁷, R⁸ = H or a substituted or unsubstituted, aliphatic or aromatic hydrocarbon radical having from 1 to 25 carbon atoms, where R⁷ and R⁸ are identical or different,
M = an alkali metal ion, alkaline earth metal ion, ammonium ion or phosphonium ion,
Q = a hydrocarbon radical of the formula IV 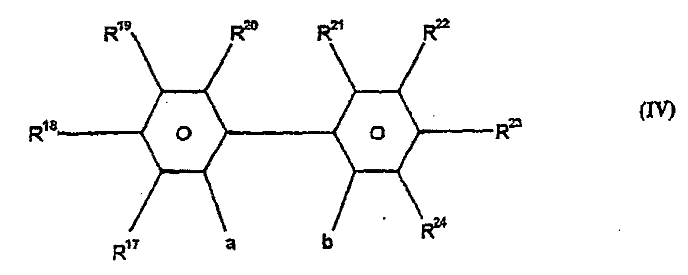 where
R¹⁷, R¹⁸, R¹⁹, R²⁰, R²¹, R²², R²³, R²⁴ = H, an aliphatic, alicyclic, aliphatic-alicyclic, heterocyclic, aliphatic-heterocyclic, aromatic or aliphatic-aromatic hydrocarbon radical having from 1 to 50 carbon atoms, F, Cl, Br, I, -OR²⁵, -COR²⁵, -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, where R¹⁷ to R²⁴ are identical or different and may be covalently linked to one another,
R²⁵, R²⁶ = H or a substituted or unsubstituted, aliphatic or aromatic hydrocarbon radical having from 1 to 25 carbon atoms,
M = an alkali metal ion, alkaline earth metal ion, ammonium ion or phosphonium ion,
where the positions a and b serve as linkage points,
W, X = aliphatic, alicyclic, aliphatic-alicyclic, heterocyclic, aliphatic-heterocyclic, aromatic or aliphatic-aromatic hydrocarbon radicals having from 1 to 50 carbon atoms, which may be identical or different or may be covalently linked to one another.

5. A bisphosphite-metal complex according to claim 4, **characterized in that**
W and X are aliphatic, alicyclic, aliphatic-alicyclic, heterocyclic, aliphatic-heterocyclic, aromatic or aliphatic-aromatic hydrocarbon radicals which have from 1 to 50 carbon atoms and are covalently linked as in the formula II 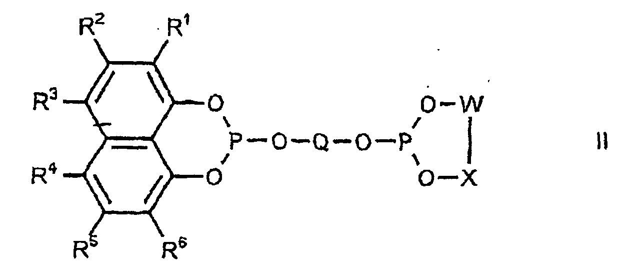 and R¹, R², R³, R⁴ , R⁵, R⁶ and Q are as defined in claim 1.

6. A bisphosphite-metal complex according to claim 4, **characterized in that**
W and X are aromatic hydrocarbon radicals which have from 1 to 50 carbon atoms and are covalently linked as in the formula III 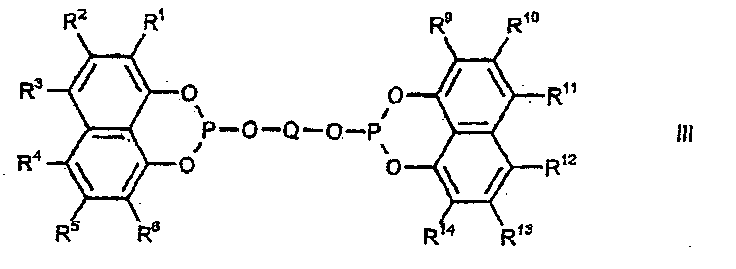 where R⁹, R¹⁰, R¹¹, R¹², R¹³, R¹⁴ = H, an aliphatic, alicyclic, aliphatic-alicyclic, heterocyclic, aliphatic-heterocyclic, aromatic or aliphatic-aromatic hydrocarbon radical having from 1 to 50 carbon atoms, F, Cl, Br, I, -OR¹⁵, -COR¹⁵, -CO₂R¹⁵, -CO₂M, -SR¹⁵, -SO₂R¹⁵, -SOR¹⁵, -SO₃R¹⁵, -SO₃M, -SO₂NR¹⁵R¹⁶, NR¹⁵R¹⁶, N=CR¹⁵R¹⁶, NH₂, where R⁹ to R¹⁴ are identical or different and may be covalently linked to one another,
R¹⁵, R¹⁶ = H or a substituted or unsubstituted, aliphatic or aromatic hydrocarbon radical having from 1 to 25 carbon atoms, where R¹⁵ and R¹⁶ are identical or different,
M = an alkali metal ion, alkaline earth metal ion, ammonium ion or phosphonium ion and
R¹, R², R³, R⁴, R⁵, R⁶ and Q are as defined in claim 1.

7. A bisphosphite-metal complex according to any one of claims 4 to 6,
**characterized in that**
the metal used is rhodium, platinum, cobalt or ruthenium.

8. The use of a bisphosphite according to any one of claims 1 to 3 in a process for the hydroformylation of olefins.

9. The use of a bisphosphite-metal complex according to any one of claims 4 to 7 in a process for the hydroformylation of olefins.

10. The use of a bisphosphite according to any one of claims 1 to 3 in a process for the hydroformylation of olefins in the presence of further phosphorus-containing ligands.

11. The use of a bisphosphite-metal complex according to any one of claims 4 to 7 in a process for the hydroformylation of olefins in the presence of further phosphorus-containing ligands.

## Revendications

1. Bisphosphite de formule I : 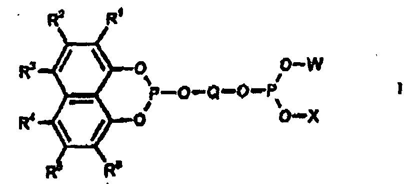 avec R¹, R², R³, R⁴, R⁵, R⁶ = H, reste hydrocarboné aliphatique, alicyclique, aliphatico- alicyclique, hétérocyclique, aliphatico- hétérocyclique, aromatique, aliphatico- aromatique, ayatn de 1 à 50 atomes de carbone, F, Cl, Br, I, -OR⁷, -COR⁷ -CO₂R⁷- CO₂M, SR⁷, -SO₂R⁷, -SOR⁷R⁷, SO₃R⁷, SO₃M, -SO₂NR⁷ R⁸, NR⁷R⁸, N = CR⁷ R⁸, NH₂, dans laquelle R¹ à R⁶ possèdent une signification identique ou différente et peuvent être couplés d'une manière covalente ;
R⁷, R⁸ = H, reste hydrocarboné substitué ou non substitué aliphatique ou aromatique ayant de 1 à 25 atomes de carbone ayatn une signification identique ou différente ;
M = ion de métal alcalin, de métal alcalino terreux d'ammonium, de phosphonium ;
O = un reste hydrocarboné conformément à la formule IV : 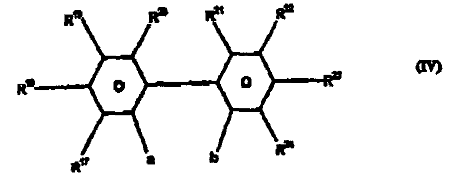 avec R¹⁷, R¹⁸, R¹⁹, R²⁰, R²¹, R²², R²³ et R²⁴ = H, reste hydrocarboné aliphatique, alicyclique, aliphatico- alicyclique, hétérocyclique, aliphaticohétérocyclique, aromatique, diphatico- aromatique, ayant de 1 à 50 atomes de carbone, F, Cl, Br, I, OR²⁵, -COR²⁵ -CO₂R²⁵ - CO₂M, SR²⁵, -SO₂R²⁵, -SOR²⁵, SO₃R²⁵, SO₃M, -SO₂NR⁷ R²⁶, NR²⁵R²⁶, N = CR²⁵ R²⁶, NH₂, où R¹⁷ à R²⁴ possèdent une signification identique ou différente et peuvent être couplés d'une manière covalente ;
R²⁵, R²⁶ = H un reste hydrocarboné substitué ou non substitué, aliphatique ou aromatique ayant de 1 à 25 atomes de carbone ;
M = ion de métal alcalin, de métal alcalino terreux, d'ammonium, de phosphonium,
dans lesquels les positions a et b servent comme position de couplage ;
W, X = restes hydrocarbonés aliphatiques, alicycliques, aliphatico- alicycliques, hétérocycliques, aliphatico- hétérocycliques, aromatiques, aliphatico- aromatiques ayant de 1 à 50 atomes de carbone, qui peuvent être identiques ou différents ou être couplés les uns avec les autres d'une manière covalente.

2. Bisphosphite conformément à la revendication 1,
**caractérisé en ce que**
W et X sont des restes hydrocarbonés aliphatiques, alicycliques, aliphatico- alicycliques, hétérocycliques, aliphatico- hétérocycliques, aromatiques, aliphatico- aromatiques, ayant de 1 à 50 atomes de carbone, ayatn un couplage covalent, répondant à la formule II : 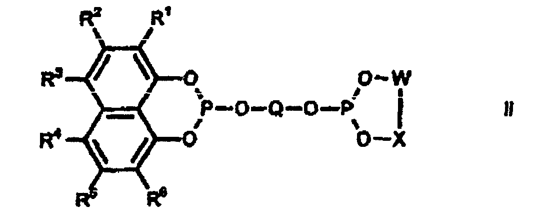 et R¹, R², R³, R⁴, R⁵, R⁶ et Q possèdent les significations mentionnées à la revendication 1.

3. Bisphosphite conformément à la revendication 1,
**caractérisé en ce que**
W et X sotn des restes hydrocarbonés aromatiques ayant de 1 à 50 atomes de carbone présentant des couplages covalents conformément à la formule III : 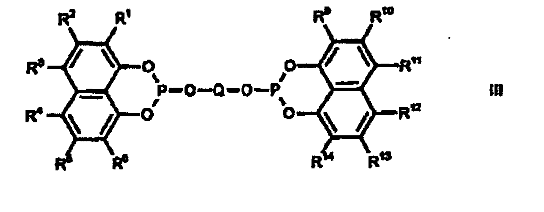 avec R⁹, R¹⁰, R¹¹, R¹², R¹³, R¹⁴ = H un reste aliphatique, alicyclique, aliphatico- alicyclique, héterocyclique, aliphatico- hétérocyclique, aromatiques, aliphatico- aromatique, ayant de 1 à 50 atomes de carbone, F, Cl, Br, I, OR¹⁵, -COR¹⁵ -CO₂R¹⁵ - CO₂M, SR¹⁵, -SO₂R¹⁵, -SOR¹⁵, SO₃R¹⁵, SO₃M, -SO₂NR¹⁵ R¹⁶, NR¹⁵R¹⁶, N = CR¹⁵ R¹⁶, NH₂, dans lesquels R9 à R¹⁴ possèdent une signification identique ou différente et peuvent être couplés les uns avec les autres d'une manière covalente ;
R¹⁵, R¹⁶ = H, reste hydrocarboné substitué ou non substitué, ayant de 1 à 25 atomes de carbone, présentant une signification identique ou différente ;
M = ion de métal alcalin, de métal alcalino terreux, d'ammonium, de phosphonium et R¹, R² , R³, R⁴, R⁵, R⁶ et Q possèdent les significations mentionnées à la revendication 1.

4. Complexe bisphosphite métal contenant un métal du 4^{ème}, 5^{ème}, 6^{ème}, 7^{ème} ou 8^{ème} sous groupe du système périodique des éléments et un ou plusieurs bisphosphite(s) de formule I : 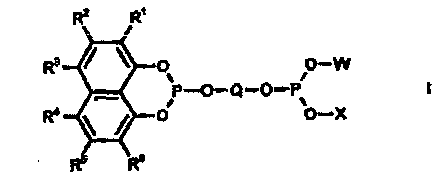 avec R¹, R², R³, R⁴, R⁵, R⁶ = H, reste aliphatique, alicyclique, aliphatico-alicyclique, hétérocyclique, aliphatico- hétérocyclique, aromatique aliphatico- aromatique, ayant de 1 à 50 atomes de carbone, F, Cl, Br, I, OR⁷, -COR⁷ -CO₂R⁷ - CO₂M, SR⁷, -SO₂R⁷, -SOR⁷, SO₃R⁷, SO₃M, -SO₂NR⁷ R⁸, NR⁷R⁸, N = CR⁷ R⁸, NH₂, où R¹ à R⁶ possèdent une signification identique ou différente et peuvent être couplés les uns avec les autres d'une manière covalente ;
R⁷, R⁸ = H, reste hydrocarboné substitué ou non substitué, aliphatique ou aromatique ayant de 1 à 25 atomes de carbone, ayant une signification identique ou différente ;
M = ion de métal alcalin, de métal alcalino terreux, d'ammonium, de phosphonium **;**
Q un reste hydrocarboné conformément à la formule IV : 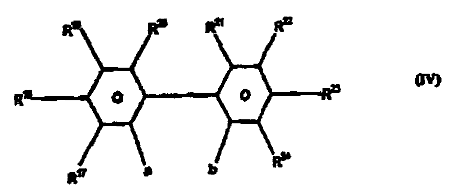 avec R¹⁷, R¹⁸, R¹⁹, R²⁰, R²¹, R²², R²³ et R²⁴ = H, reste hydrocarboné aliphatique, alicyclique, aliphatico- alicyclique, hétérocyclique, aliphatico-hétérocyclique, aromatique, diphatico- aromatique, ayant de 1 à 50 atomes de carbone, F, Cl, Br, J, OR²⁵, -COR²⁵ -CO₂R²⁵ - CO₂M, SR²⁵, -SO₂R²⁵, -SOR²⁵, SO₃R²⁵, SO₃M, -SO₂NR²⁵ R²⁶, NR²⁵R²⁶, N = CR²⁵ R²⁶, NH₂, où R¹⁷ à R²⁴ possèdent une signification identique ou différente et peuvent être couplés les uns aux autres d'une manière covalente ;
R²⁵, R²⁶ = H, un reste hydrocarboné substitué ou non substitué, aliphatique, ou aromatique ayant de 1 à 25 atomes de carbone,
M = ion de métal alcalin, de métal alcalino terreux, d'ammonium, de phosphonium, où les positions a et b servent comme points de couplage ;
W, X = resstes hydrocarbonés aliphatiques, alicycliques, aliphatico- alicycliques, hétérocycliques, aliphatico- hétérocycliques, aromatiques, aliphatico- aromatiques, ayant de 1 à 50 atomes de carbone qui peuvent être identiques ou différents ou être couplés les uns avec les autres d'une manière covalente.

5. Complexe de métal de bisphosphite selon la revendication 4,
**caractérisé en ce que**
W et X sont des restes hydrocarbonés aliphatiques, alicycliques, aliphatico- alicycliques, hétérocycliques, aliphatico- hétérocycliques, aromatiques, aliphatico aromatiques, ayant de 1 à 50 atomes de carbone, présentant un couplage covalent conformément à la formule II : 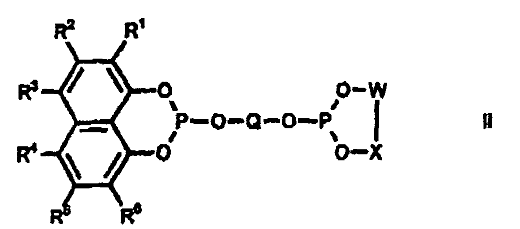 et R¹, R² , R³, R⁴, R⁵, R⁶ et Q possèdent les significations mentionnées à la revendication 1.

6. Complexe de métal de bisphosphite selon la revendication 4,
**caractérisé en ce que**
W et X sont des restes hydrocarbonés aromatiques ayant de 1 à 50 atomes de carbone présentant des couplages covalents conformément à la formule III : 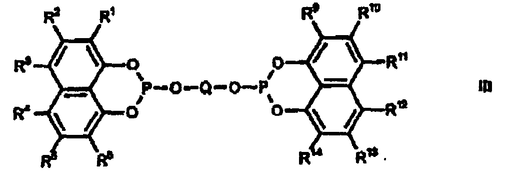 avec R⁹, R¹⁰ , R¹¹, R¹², R¹³ et R¹⁴ = H, reste hydrocarboné aliphatique, alicyclique, aliphatico- alicyclique, hétérocyclique, aliphatico- hétérocyclique, aromatique, aliphatico- aromatique, ayant de 1 à 50 atomes de carbone, F, Cl, Br, I, OR¹⁵, -COR¹⁵ -CO₂R¹⁵ - CO₂M, SR¹⁵, -SO₂R¹⁵, -SOR¹⁵, SO₃R¹⁵, SO₃M, -SO₂NR¹⁵ R¹⁶, NR¹⁵R¹⁶, N = CR¹⁵ R¹⁶, NH₂, où R⁹ à R¹⁴ possèdent une signification identique ou différente et peuvent être couplés les uns avec les autres d'une manière covalente ;
R¹⁵, R¹⁶ = H, reste hydrocarboné substitué ou non substitué, aliphatique ou aromatique, ayant de 1 à 25 atomes de carbone, présentant une signification identique ou différente ;
M = un ion de métal alcalin, de métal alcalino terreux, d'ammonium, de phosphonium, et R¹, R², R³, R⁴, R⁵, R⁶ et Q possèdent les significations mentionnées à la revendication 1.

7. Complexe métallique de bisphosphite selon l'une des revendications 4 à 6,
**caractérisé en ce qu'**
on met en oeuvre comme métal, du rhodium, du platine, du cobalt ou du ruthénium.

8. Utilisation des bisphosphites conformément à l'une des revendications 1 à 3, dans un procédé d'hydroformulation d'oléfines.

9. Utilisation des complexes métalliques de bisphosphite conformément à l'une des revendications 4 à 7,
dans un procédé d'hydroformylation d'oléfines.

10. Utilisation des bisphosphites conformément à l'une des revendications 1 à 3, dans un procédé d'hydroformylation d'oléfines en présence d'autres ligands contenant du phosphore.

11. Utilisation des complexes métalliques de bisphosphite conformément à l'une des revendications 4 à 7, dans un procédé d'hydroformylation d'oléfines en présence d'autres ligands contenant du phosphore.
